# EUROPEAN PATENT APPLICATION

(11) **EP 3 985 680 A1**
(43) Date of publication of application: **20.04.2022**
(21) Application number: 20201429.6
(22) Date of filing: 13.10.2020
(51) Int. Cl.: G16H 20/10, G16H 20/70

(54) **SYSTEM AND METHOD FOR PERSONALIZATION OF SLEEP RESTRICTION THERAPY**

(71) Applicant: Koninklijke Philips N.V., 5656 AG Eindhoven (NL)
(72) Inventor: WESTERINK, Joanne Henriëtte Desirée Monique, 5656 AE Eindhoven (NL); MENA BENITO, Maria Estrella, 5656 AE Eindhoven (NL); LEUFKENS, Timmy Robertus Maria, 5656 AE Eindhoven (NL); VAN EE, Raymond, 5656 AE Eindhoven (NL); PFUNDTNER, Stefan, 5656 AE Eindhoven (NL); WEYSEN, Tim, 5656 AE Eindhoven (NL)
(74) Representative: Philips Intellectual Property & Standards

(57) **Abstract**

A system for guiding sleep restriction therapy is based on sleep data as well as medication data for a subject for a plurality of nightly sleep sessions. The sleep data and the medication data are processed to derive a target in-bed duration and to derive a target medication dose. A sleep restriction and medication recommendation are then output based on the target in-bed duration and the target medication dose. This system implements an improved sleep restriction therapy method that not only promotes the usual gradual increase of the allowed sleeping window, but also enables implementation of a gradual decrease of sleeping medication, which is beneficial for therapy adherence.

## Description

### FIELD OF THE INVENTION

This invention relates to sleep restriction therapy, and in particular it relates to a system for automating the decision making in sleep restriction therapy.

### BACKGROUND OF THE INVENTION

Insomnia is defined as the perception or complaint of inadequate or poor-quality sleep due to a number of factors, such as difficulty falling asleep, waking up frequently during the night with difficulty returning to sleep, waking up too early in the morning, or unrefreshing sleep. It is one of the most prevalent sleep disorders in the world, since it is generally believed that 10% to 15% of the adult population suffers from chronic insomnia, and an additional 25% to 35% have transient or occasional insomnia.

Insomnia is typically treated using pharmacologic therapies. The article "A behavioral perspective on insomnia treatment", Spielman AJ, Caruso LS, Glovinsky PB., Psychiatr. Clin. North Am. 1987;10:541 describes, a so-called 3P-model which concludes that the use of sleep medication might in fact have detrimental effects in the long run, leading to chronic insomnia.

The 3P model is based on 3 factors that determine the extent of sleeping problems (predisposing factors, precipitating factors and perpetuating factors).

The predisposing factors constitute the natural disposition for sleeping well or sleeping badly that individuals are born with.

The predisposing factors in themselves are not generally the reason that people turn to using sleeping medication, as their level of sleeping problems (insomnia severity) in a preclinical stage is often considerably below an insomnia threshold.

The precipitating factors constitute negative life events that make people worry and ruminate at night. This will indeed lead to insufficient sleep quality for the duration of the negative feelings. The precipitating factors however have a reversible impact on sleep quality: when the worries subside, sleep quality improves. However, for various reasons, many people cannot wait until that happens, and they turn to sleep medication.

The perpetuating factors help the sleep problems to continue. Sleep medication can itself be a perpetuating factor.

At first, sleep medication helps to relieve the sleep problems somewhat, but the quality of the sleep is reduced. A consequence is that, even if the perpetuating factors have subsided, sleeping problems remain, possibly above an insomnia threshold. At this point, the insomnia has become a chronic problem.

During recent decades, non-pharmacologic behavioral therapies have been shown to be effective for the treatment of insomnia. They have many advantages over pharmacologic therapies, including no risk of tolerance issues or dependency, and reduced side effects. They work on correcting the route cause instead of treating the associated symptoms. Examples of behavioral therapies include: stimulus control therapy, sleep restriction therapy and relaxation training.

Sleep restriction therapy is often used as part of cognitive behavioral therapy. Sleep restriction therapy is a very effective behavioral treatment for insomnia that works to decrease variability in the timing of sleep while increasing the depth of sleep. The goal is to shorten the amount of time spent in bed in order to consolidate sleep. It takes however several weeks of diligent dedication to altering a subject's sleep schedule in order to observe improvements in sleep. Subjects feel sleepier and experience more disrupted sleep initially, but the eventual beneficial effects are long lasting.

Sleep restriction therapy generally incorporates the following steps:
Step 1: Determine an allowed time in bed. This begins by allowing staying in bed for only the average amount of time a subject (i.e. a sleep therapy patient) is actually currently sleeping. This can be calculated by keeping a sleep diary for a time period, such as two weeks. The average total sleep time for the monitoring period is determined and 30 minutes is added to derive an allowed time in bed.
Step 2: Set a wake time. The wake up time is set at the same time every morning no matter how much sleep the subject had the night before.
Step 3: Set a go to bed time. The go to bed time is determined by counting back from a desired wake time up by the allowed time in bed set in Step 1. The subject should not get into bed before the go to bed time, even if the subject is sleepy and believes they could fall asleep.
Step 4: Stick to the sleep schedule as closely as possible for at least two weeks.

Thus, the sleep restriction therapy involves allowing the subject to stay in bed no longer than the number of hours of sleep get on average based on analysis of a sleep diary. Although in the first nights the sleep will be very fragmented, sleep pressure will build up within a few days, resulting in better sleep in subsequent nights. If this is achieved, the number of hours allowed in bed (the length of the sleeping window) is gradually increased, until the intended duration of good quality sleep is reached. This therapy is often coached by sleep therapists who work at sleep clinics. It can also be implemented in the form of a mobile phone app.

While sleep restriction therapy is known to work when adhered to, many people are very anxious about not getting the "usually prescribed" 8 hours of sleep each night. They fear that the therapy will make them function and feel even worse. It is one of the reasons why subjects often fail to adhere to the therapy, which then reduces its beneficial effects. As a result, the sleep restriction therapy may in fact lead subjects to use sleeping medication as well or instead.

It is possible for patients to stop taking sleep medication before starting the sleep therapy, for example to obtain accurate baseline sleep data. However, allowing patients to initiate sleep therapy without discontinuing medication may increase referrals willing to engage in the therapy. Patients may then discontinue medication during the therapy. They may then experience rebound insomnia symptoms but they will then receive support and encouragement to continue with the sleep therapy.

Existing sleep restriction therapy approaches do not take account if the user is using sleep medication.

### SUMMARY OF THE INVENTION

The invention is defined by the claims.

According to examples in accordance with an aspect of the invention, there is provided a processor for a system for sleep restriction therapy, comprising:
a first input for receiving sleep data for a subject for a plurality of nightly sleep sessions, which indicates at least times during a sleep session that the subject is asleep and times during the sleep session that the subject is awake;
a second input for receiving medication data for said subject for said plurality of nightly sleep sessions, which indicates medication taken by the subject;
wherein the processor is adapted to process the sleep data and the medication data to:
   derive a target in-bed duration;
   derive a target medication dose; and
   output a sleep restriction and medication recommendation based on the target in-bed duration and the target medication dose.

This system implements an improved sleep restriction therapy method that not only promotes the usual gradual increase of the duration of the allowed sleeping window, but also enables implementation of a gradual decrease of sleeping medication. This has a number of advantages. First, the therapy explicitly takes the sleep medication into account, which means that the subject has less reason to be secretive about the use of sleep medication. Furthermore, allowing patients to initiate the sleep restriction therapy without discontinuing medication may increase referrals willing to engage. Knowing about the presence of this perpetuating factor can increase the chance of success of the sleep restriction therapy.

Secondly, the system provides recommendations which can lead to an optimal sleep pattern without the use of sleep medication, which is healthier in the long run. Thirdly, subjects can be explicitly coached on the reduction of their sleep medication intake. In this way, anxiety concerning the impossibility of falling asleep without sleep medication can be addressed. Moreover, this can be done gradually, so that anxiety is reduced and trust is built up in small steps.

The invention thus provides an improved sleep restriction therapy method that enables gradual increase of the duration of the allowed sleeping window in combination with gradual decrease of sleeping medication.

The target medication dose may be a dose of a medication that the subject is already taking, or it may be a dose of a new medication (e.g. to replace a previous stronger medication that the subject is taking). The target medication dose may be zero.

The processor receives sleep data for a plurality of sleep sessions. One sleep session is one period of intended sleep, i.e. one night. The actual sleep performance of the subject is analyzed over a sequence of sleep sessions (i.e. nights) and from this analysis a sleep restriction recommendation and medication recommendation is generated.

The sleep data is for example based on self-reporting by the subject, for example by indicating for a sequence of epochs during the night whether they were asleep or awake, which is a common method to evaluate a subject's sleep in a sleep diary.

The sleep restriction recommendation may be used to control a system which provides a bed time indicator and a wake up time indicator. Such a system may be a basic notification and/or alarm or it may be a more elaborate sleep control system (e.g. which controls the lighting and/or sound at bed time and controls the lighting and/or sound at wake up time).

The processor may for example be part of a mobile phone or tablet on which a suitable app has been loaded. The processor may be part of an output device by which the sleep restriction recommendation is provided to the subject or clinician, or it may be a different device.

The medication data for example comprises a medication type, medication dose and medication timing, which is recorded by daily self-reports, completed by the subject.

The processor may further comprise a third input for receiving impact data from a data structure which contains data relating to the impact of different doses of different sleep medications on sleep characteristics for the general population. By knowing expected impact of sleep medication, it becomes possible to provide medication recommendations which achieve the desired gradual change in sleeping characteristics over time, when combined with recommended sleep time windows.

The processor may be further adapted to derive personalized impact data relating to the impact of different doses of different sleep medications on sleep characteristics of the particular subject using the system. Thus, the particular effect of medications on the particular subject can be taken into account, by learning from historical data for that subject.

Additionally, the system may create impact data for a particular subject based on data of comparable other subjects, when no historical data is available for a particular subject.

The plurality of sleep sessions for example comprises between 7 and 21 sleep sessions. Thus, the sleep pattern may be analyzed for one to three weeks before a sleep restriction recommendation is generated.

The processor may derive the target in-bed duration based at least on an estimate of the average total actual sleep time for the plurality of sleep sessions. The sleep restriction recommendation is modified over time based on new sleep data received over time. The allocated time in bed thus corresponds to the actual amount of sleep the subject generally achieves. This is the basic operation of a sleep restriction therapy approach.

The go to bed time may be set never to be earlier than the actual fall asleep time which has been observed historically. Thus the subject should be ready for sleep when they go to bed.

The processor may be further adapted to modify the sleep restriction recommendation over time based on new sleep data and medication data received over time. Thus, the progression over time of a sleep restriction therapy may also be handled by the system based on the continuously collected sleep data.

The sleep data for example comprises subject input. Sleep questionnaires/diaries can be used to provide at least the initial sleep data. Thus, self-reporting of the sleep performance during a preceding night may be used as the primary source of data. This is typically the case for sleep restriction therapy. The probable over-exaggeration of lack of sleep which is typical in self-reported sleep data is factored into the analysis of the data.

The sleep data may optionally also comprise sensor data. Sensor data may supplement the self reporting data, and may for example for of interest for collecting data in the middle of a sleep session. It may also allow shorter time epochs to be used.

The medication data may also comprise subject input. The subject for example simply needs to input to the system the type and dose of medication taken, ideally at the time the medication is taken (or else the time will additionally need to be input).

The medication data may include data from an electronic medication dispensing system so that the data may be collected at least partly automatically.

The processor may further comprise an input for receiving sleep preferences of the subject, wherein the processor is adapted to derive the target timing taking into account the sleep preferences.

The sleep preferences for example may relate to latest times the subject can stay in bed (e.g. because of work commitments), so that the wake up time has a latest possible time point.

The invention also provides a system for sleep restriction therapy, comprising:
a sensor arrangement for collecting sensor data and/or medication data; and
the processor as defined above for processing the sensor data and medication data to generate the sleep restriction and medication recommendation.

In this way, a system includes sensors to supplement the self-reporting data.

The sensor arrangement may comprise a single sensor unit or a system of sensor units. The sensor arrangement may comprise at least one body-worn sleep sensor. This may be a head band or a wrist band. A head band for example enables EEG monitoring, and such sleep tracking headband devices are well known. PPG and motion monitoring using a wrist watch type device is also well known for providing sleep data.

The sensor arrangement may instead or additionally comprise at least one monitoring sensor remote from the subject. This may for example monitor movements, but also provide additional environmental information of interest. This sensor arrangement may for example report times when lights are turned on or off, or provide temperature monitoring (to sense the presence of a body) etc. These may provide additional data for verifying or enhancing the self reporting data or data from body-worn sensors.

The sensor arrangement may instead or additionally comprise a medication dispensing system. This may automatically provide the medication data.

The invention also provides a computer-implemented method of generating a sleep restriction recommendation and medication dose recommendation for sleep restriction therapy, comprising:
receiving sleep data for a subject for a plurality of nightly sleep sessions, which indicates at least time epochs during a sleep session that the subject is asleep and time epochs during the sleep session that the subject is awake;
receiving medication data for said subject for said plurality of nightly sleep sessions, which indicates medication taken by the subject;
deriving a target in-bed duration;
deriving a target medication dose; and
output a sleep restriction and medication recommendation based on the target in-bed duration and the target medication dose.

This method takes account of both the sleep patterns of a subject and the medication history, in order to structure a sleep restriction therapy.

The method may comprise receiving data from a data structure which contains impact data relating to the impact of different doses of different sleep medications on sleep characteristics for the general population, and taking account of the impact data when deriving the target in-bed duration and target medication dose.

This allows the effects of medication to be taken into account.

The method may further comprise deriving personalized impact data relating to the impact of different doses of different sleep medications on sleep characteristics of the particular subject using the system.

The invention also provides a computer program comprising computer program code which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a system for sleep restriction therapy; and
Figure 2 shows the method implemented by the processor of Figure 1.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for guiding sleep restriction therapy based on sleep data as well as medication data for a subject for a plurality of nightly sleep sessions. The sleep data and the medication data are processed to derive a target in-bed duration and to derive a target medication dose. A sleep restriction and medication recommendation are then output based on the target in-bed duration and the target medication dose. This system implements an improved sleep restriction therapy method that not only promotes the usual gradual increase of the allowed sleeping window, but also enables implementation of a gradual decrease of sleeping medication, which is beneficial for therapy adherence.

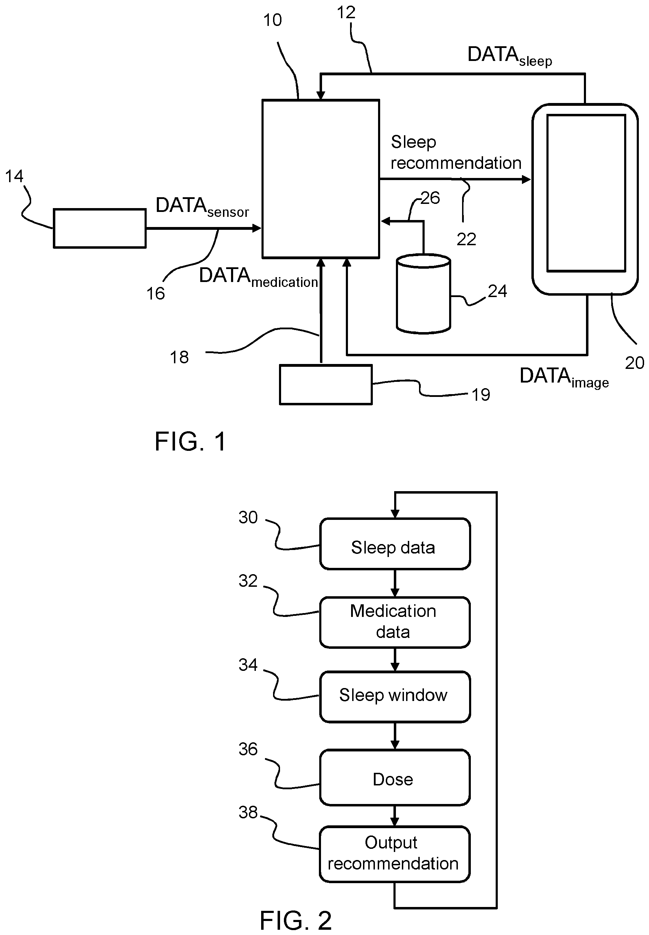

Figure 1 shows a system for sleep restriction therapy, comprising a processor 10 and a user interface device 20. The user interface device is a device for providing instructions to the subject, such as an alarm clock which may set both a go to bed time and a wake up time. The user interface device may simply comprise a mobile phone or tablet on which suitable software is loaded, and the processor 10 may then be the processor of that device. Alternatively, the processor may be part of a separate device which then communicates with the user interface device 20.

The processor has an input 12 for receiving sleep data DATAₛₗₑₑₚ for a subject for a plurality of nightly sleep sessions, which indicates at least times, such as epochs, during a sleep session that the subject is asleep and times, such as epochs, during the sleep session that the subject is awake. The processor includes a memory (database) for storing the data for subsequent analysis.

This sleep data is for example based on a self-reporting sleep report such as a sleep diary which indicates for a sequence of epochs during the night whether they were asleep or awake. Such a sleep diary may be based on a digital sleep/wake diary maintained by the subject for self-reporting of their sleep/awake timings. The reporting to the diary could be achieved using a user interface to a digital diary, for example by means of a dedicated app on their smartphone or tablet, or an existing app associated with the user interface device 20.

The diary is for example maintained for a period of sleep 1 or 2 weeks prior to the intervention.

Optionally, a sensor arrangement 14 is provided for collecting sensor data DATAₛₑₙₛₒᵣ. The processor additionally processes the sensor data. The sensor arrangement is for example used to supplement the self-reporting data. The sensor arrangement 14 may comprise at least one body-worn sleep sensor. This may be a head band or a wrist band. A head band for example enables EEG monitoring, and such sleep tracking headband devices are well known. PPG and motion monitoring using a wrist watch type device is also well known for providing data indicating sleep information. Less obtrusive sensors may also be used, for example cameras, pillow-based sensors or mattress-based sensors.

The system also receives medication data DATA_{medication} 18. This allows the system to know the amount, the time and the type of sleep medication the subject is taking.

The data could be derived from an on-line questionnaire to be filled out by the subject using the user interface device 20, in which the current and past use of sleep medication is detailed. Each morning the subject could be requested to update the type and dose of sleep medication taken in the past 24 hours, and when exactly.

The system may additionally or alternatively be linked to an electronic sleep medication dispenser 19, so that the amount of sleep medication taken each day is automatically entered in the system.

Another option is for the subject using the system to use a camera function of the user interface device 20 to scan a label or barcode of the medication package and provide image data DATA_{image} to the processor, so again the medication information is automatically entered into the system. The timing may be inferred from the time the image is uploaded to the system and/or the subject may manually enter the timing and optionally also dose (if there are different dose options).

The processor 10 processes the sleep data and the medication data to derive a target in-bed duration. Optionally, it may also derive a target timing for the target in-bed duration, i.e. the time to go to bed and the time to get up. The target in-bed duration and the time to go to bed together define a sleep window. The timing is for example selected to achieve the most sleep during the in-bed duration. Alternatively, the subject may choose a time to go to bed or a time to get up.

A sleep restriction and medication dose recommendation 22 is then generated based on the target in-bed duration and the target timing, and on a determined medication dose. This is provided to the user interface device 20.

The processor 10 receives sleep data DATAₛₗₑₑₚ for a plurality of sleep sessions. One sleep session is one period of intended sleep, i.e. a time in bed during one night. The actual sleep performance of the subject is analyzed over a sequence of sleep sessions (i.e. nights) and from this analysis the sleep restriction recommendation is generated.

Various examples of how the data is processed will now be explained.

Figure 2 shows a most basic example, in which there are five steps carried out by the system each day or every set of days, e.g. every other day.

In step 30, the sleep data and sensor data are provided to the system. From this, the system derives the sleep duration in the past few nights from the data generated by the sleep data input or by the input from the sensor arrangement 14. For example, the subject might have indicated that while they are in bed for a duration of 9 hours per night, they only sleep 5 hours per night.

In step 32, the system determines from the medication data DATA_{medication} the strength of the sleep medication taken in the past few days. For example, the subject might have indicated that each night they take 30 mg of Temazepam before going to bed.

In step 34, the system proposes a sleeping window for the upcoming nights, on the basis of the average amount of hours slept in the last few nights as well as the medication data. The sleeping window defines the length (duration) and start time of the sleep session.

In step 36, the system proposes a medication dose for the upcoming nights, again on the basis of the average amount of hours slept in the last few nights as well as the medication data.

For the above subject, the advised sleeping window length will for example be set to 5 hours. Thus the subject might be advised to go to bed at 1 o'clock at night, and rise at 6 o'clock in the morning, even if the subject does not sleep all of these 5 hours. The aim is that the subject will become sleepy because they do not sleep enough (which they did not do anyway), and because of that after a while they will at least sleep with less interruptions.

If after a few days, the sleep quality (averaged over a few nights) in the sleeping window increases, the system will then propose to increase the length of the sleeping window.

For example, the above subject may sleep most of the 5 hours of the sleeping window after a while. The system will then advise to lengthen the sleeping window, for instance to 5 hours 15 minutes, starting at 12:45am and still rising at 6 o'clock in the morning. When the 5 and a quarter hour sleeping window is successful as well, the length of the sleeping window can be increased again, and again, until an acceptable sleeping window length is reached. The time increments may be 15 minutes as in this example but there may be other time increments.

Instead of (or in addition to) increasing the length of the sleeping window, the system of the invention can also propose to decrease the dose of sleep medication for the upcoming few nights. For example, when the subject has just successfully adapted to a sleeping window length of 5.5 hours in the past few days, it could be decided not to increase the sleeping window length again, but instead reduce the sleeping medication dose taken each night.

Then, a reduced amount of sleep medication, for example 66% of the current dose (20 mg of Temazepam), is advised to the subject for the upcoming days. Again, it is expected that the subject will sleep less, but if so, they will become sleepier during the day, resulting over time in better sleep even with the lower sleep medication dose. If this happens, it can then be decided to again increase the sleeping window length or reduce the sleep medication dose further, until eventually an acceptable sleeping window length without sleep medication is reached.

The duration adaptation is the standard evolution of the sleep restriction therapy. The invention incorporates medication dosage levels into the sleep recommendations provided to the subject, with the aim of gradually decreasing the sleep medication while at the same time increasing the sleep window to a desired steady state level.

There are various ways in which the medication data can be taken into account. One approach is to make use data structures such as look up tables which encode a sleep medication effect/impact for each dose of each common type of sleep medication.

Figure 1 shows a data structure a data structure 24 which contains data relating to the impact of different doses of different sleep medications on sleep characteristics. The data is provided to a third input 26 of the processor 10.

Such impacts may be known from data on the general (insomnia) population. Thus, the system has a data structure which contains data relating to the impact of different doses of different sleep medications on sleep characteristics. The system may of course instead access a remote database which stores this information.

On the basis of the types and doses taken in the past few nights, the processor may then calculate an average effect/impact.

In order to change the reliance on medication, the current impact of the medication being taken is determined. A certain percentage of that effect/impact is then taken, e.g. to reduce the beneficial effect to 80% of its previous value. The required medication type and dose for this reduced beneficial effect is then found from the data structure, and this new type and dose is provided as the new guidance for the upcoming nights. Thus, the medication type and medication dose may both be adjusted as the system weans the subject off the medication in a gradual manner, while at the same time (either alternately or even together) increasing the in bed time as the therapy progresses.

A calculation using such a data structure is of particular benefit if different types of medication are used by the subject, either alternating over nights or in combination in a single night, for instance Temazepam and alcohol. The data structure could therefore also contain information about the effect/impact of combinations of medication in various doses.

On the basis of the sleep data and medication data, the system may also derive the effect/impact of the sleep medication on the particular individual subject. For example, the system could detect that a 20 mg dose of Temazepam leads on average to 4 hours of sleep, whereas a 30 mg dose leads to 4.5 hours of sleep on average. This enables the system to create a personalized version of the above data structure, and hence a personalization of the sleep therapy advice given.

In a similar way, the system may determine from the sleep data in which part of the night the sleeping window is most effective for this subject. For example, the system could detect that the total sleep duration is more, or the sleep fragmentation is less, when the subject goes to bed relatively late (e.g. 1.15 am in stead of 1.00 am), than when the subject goes to bed relatively early (e.g. at 0.45 am). The system could then advise that the go to bed time should be shifted to 1.15 am, while keeping the sleeping window constant. Thus, the timing of the sleep window may be adjusted according to the historical sleep data.

The start time of the sleeping window may then also be used as an additional input parameter for the personalized data structure, allowing modeling of any interactions between sleep medication type, sleep medication dose and sleeping window start time.

The processor may also take account of sleep preferences of the subject. For example, a subject may prefer to go to bed early and get up early if the probability of success of the sleep therapy is the same. The sleep preferences for example may relate to latest times the subject can stay in bed (e.g. because of work commitments), or the earliest time the subject can go to bed (e.g. because of evening commitments).

Besides the therapeutic effects (i.e. sleep improving effects), both sleep restriction and sleep medication are associated with adverse effects in the morning (and some even in the afternoon). Adverse effects can be identified by e.g. a decrease in cognitive performance, lower alertness levels and feelings of drowsiness. The system may thus also serve as a monitoring system for tracking daytime functioning and a system to warn a person undergoing therapy. Known effects of sleep restriction and sleep medication (per type and dose) can be warned in advance, and individual response to adverse effects could be tracked and a warning system could be based on that.

A warning would for example consist of mentioning the time it will take for a certain sleep medication to be metabolized and not causing any cognitive performance decrease anymore.

The plurality of sleep sessions used at the beginning of the therapy does not have to be two weeks. More generally it may be between 7 and 21 sleep sessions. Thus, the sleep pattern may be analyzed for one to three weeks before a sleep restriction therapy recommendation is generated. Once the therapy is underway, adaptations may take place daily, every other day, or every few days. The adaptations may take longer, e.g. every week, if it takes that long to reliably establish that sleep using the current sleep duration and go-to-bed time has improved sufficiently to take the next step. The adaptation time may also be personalized, for example based on the expected improvements from historical data for comparable subjects.

The sleep restriction therapy may start with a fixed target in-bed duration, e.g. of 5 hours as explained above. However, the target in-bed duration may instead be based on an estimate of the average total actual sleep time for the plurality of sleep sessions. Thus, the starting point may be based on the actual amount of sleep the subject generally achieves. This is the basic operation of a sleep restriction therapy approach.

The go to bed time may be set never to be earlier than the actual fall asleep time which has been observed historically. Thus the subject should be ready for sleep when they go to bed.

The sleep restriction recommendation will evolve over time. The processor may thus modify the sleep restriction recommendation over time based on new sleep data received over time. Thus, the progression over time of a sleep restriction therapy may also be handled by the system based on the continuously collected sleep data.

The sensor arrangement mentioned above may include a body-worn sensor, so that the processor may for example be used to extend the functionality of a sleep monitoring headband. The sensor arrangement may instead or additionally comprise at least one monitoring sensor which is less obtrusive. This may for example monitor movements, but also provide additional environmental information of interest. This sensor arrangement may for example report times when lights are turned on or off, or provide temperature monitoring (to sense the presence of a body) etc. These may provide additional data for verifying or enhancing the self reporting data or data from body-worn sensors.

The processing of the data may be handled in various ways, either locally to the subject, or remotely. For example, data may be stored in a remote database.

The system may for example automatically generate summarizing reports to give the specialist a quick overview of the data. The clinician/doctor can review the data in a graphical interface and use this to discuss the new bedtime schedule with the subject. This may enable easy adaption of treatment to maximize individual adherence. The recommendations may thus be provided to a subject (i.e. a patient) directly or to a sleep therapist and/or other clinician. This would allow the therapist/clinician to check whether any unrealistic advice is given, and if so, the therapist could be provided with a module to alter/adapt the advice.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processor (10) for a system for sleep restriction therapy, comprising:
a first input (12) for receiving sleep data (DATAₛₗₑₑₚ) for a subject for a plurality of nightly sleep sessions, which indicates at least times during a sleep session that the subject is asleep and times during the sleep session that the subject is awake;
a second input (18) for receiving medication data (DATA_{medication}) for said subject for said plurality of nightly sleep sessions, which indicates medication taken by the subj ect;
wherein the processor (10) is adapted to process the sleep data and the medication data to:
derive a target in-bed duration;
derive a target medication dose; and
output a sleep restriction and medication recommendation based on the target in-bed duration and the target medication dose.

2. The processor of claim 1, wherein the medication data (DATA_{medication}) comprises a medication type, medication dose and medication timing.

3. The processor of claim 1, further comprising a third input (26) for receiving impact data from a data structure (24) which contains data relating to the impact of different doses of different sleep medications on sleep characteristics for the general population.

4. The processor of claim 3, further adapted to derive personalized impact data relating to the impact of different doses of different sleep medications on sleep characteristics of the particular subject using the system.

5. The processor of any one of claims 1 to 4, wherein the plurality of nightly sleep sessions comprises between 7 and 21 sleep sessions.

6. The processor of any one of claims 1 to 5, adapted to derive the target in-bed duration based at least on an estimate of the average total actual sleep time for the plurality of sleep sessions.

7. The processor of any one of claims 1 to 6, further adapted to modify the sleep restriction recommendation over time based on new sleep data and medication data received over time.

8. The processor of any one of claims 1 to 7, wherein the sleep data (DATAₛₗₑₑₚ) comprises:
subject input; and
optionally, sensor data.

9. The processor of any one of claims 1 to 8, wherein the medication data (DATA_{medication}) comprises:
subject input; and
data from an electronic medication dispensing system.

10. A system for sleep restriction therapy, comprising:
a sensor arrangement (14,19) for collecting sensor data (DATAₛₑₙₛₒᵣ) and/or the medication data (DATA_{medication}); and
the processor (10) of any one of claims 1 to 9 for processing the sensor data and medication data to generate the sleep restriction and medication recommendation.

11. The system of claim 10, wherein the sensor arrangement comprises:
at least one body-worn sleep sensor (14); and/or
a medication dispensing system (19).

12. A computer-implemented method of generating a sleep restriction recommendation and medication dose recommendation for sleep restriction therapy, comprising:
(30) receiving sleep data (DATAₛₗₑₑₚ) for a subject for a plurality of nightly sleep sessions, which indicates at least times during a sleep session that the subject is asleep and times during the sleep session that the subject is awake;
(32) receiving medication data (DATA_{medication}) for said subject for said plurality of nightly sleep sessions, which indicates medication taken by the subject;
(34) deriving a target in-bed duration;
(36) deriving a target medication dose; and
(38) outputting a sleep restriction and medication recommendation based on the target in-bed duration and the target medication dose.

13. The method of claim 12, comprising:
receiving data from a data structure which contains impact data relating to the impact of different doses of different sleep medications on sleep characteristics for the general population, and taking account of the impact data when deriving the target in-bed duration and target medication dose.

14. The method of claim 13, further comprising deriving personalized impact data relating to the impact of different doses of different sleep medications on sleep characteristics of the particular subject using the system.

15. A computer program comprising computer program code which is adapted, when said program is run on a computer, to implement the method of any of claims 12 to 14.
